# EUROPÄISCHE PATENTANMELDUNG

(11) **EP 1 110 507 A2**
(43) Veröffentlichungstag der Anmeldung: **27.06.2001**
(21) Anmeldenummer: 00126945.5
(22) Anmeldetag: 08.12.2000
(51) Int. Cl.: A61B 8/13

(54) **Verfahren sowie System zur Generierung von diagnostisch verwertbaren dreidimensionalen Ultraschallbilddatensätzen**

(30) Priorität: 21.12.1999 DE 19961652; 03.02.2000 DE 10004748
(71) Anmelder: EchoTech GmbH, 85399 Hallbergmoos (DE)
(72) Erfinder: Polz, Dr. Hans, 85456 Wartenberg (DE)
(74) Vertreter: Graf, Helmut, Dipl.-Ing.

(57) **Zusammenfassung**

Die Erfindung bezieht sich auf ein neuartiges Verfahren sowie System zur Generierung von diagnostisch verwertbaren dreidimensionalen Ultraschallbilddatensätzen.

## Beschreibung

Die Erfindung bezieht sich auf ein Verfahren gemäß Oberbegriff Patentanspruch 1 sowie auf ein System zum Durchführen dieses Verfahrens gemäß Oberbegriff Patentanspruch 6.

Ein Verfahren zur Erfassung von diagnotisch verwertbaren dreidimensionalen Ultraschallbilddatensätzen ist grundsätzlich bekannt und beispielsweise in der EP 0 865 765 beschrieben. Ein wesentlicher Gesichtspunkt dieses bekannten Verfahrens ist u.a. das freies Führen des Ultraschallkopfes während der Aufnahme der Ultraschall-Einzelbilder, d.h. während der Bild- oder Datenaquisation.

Für die Gewinnung der Positions- und Orientierungsdaten wird beim bekannten Verfahren ein elektromagnetisches Sensorsystem verwendet, welches beispielsweise unter der Bezeichnung "ISOTRAK II" von der Firma POLHEMUS, One Hercules Drive, Colchester, VT 05446 angeboten wird.

Aufgabe der Erfindung ist es, ein Verfahren sowie ein System zur Erfassung von diagnostisch verwertbaren dreidimensionalen Ultraschallbilddatensätzen dahingehend zu verbessern, daß bei Beibehaltung einer freien manuellen Führung des Ultraschallwandler bzw. des Ultraschallkopfes eine vereinfachte Ausbildung dieses Ultraschallkopfes möglich ist.

Zur Lösung dieser Aufgabe ist ein Verfahren entsprechend dem Patentanspruch 1 ausgebildet. Ein System ist entsprechend dem Patentanspruch 6 ausgebildet.

Weiterbildungen der Erfindung sind Gegenstand der Unteransprüche. Die Erfindung wird im Folgenden anhand der Figuren an einem Ausführungsbeispiel näher erläutert. Es zeigen:

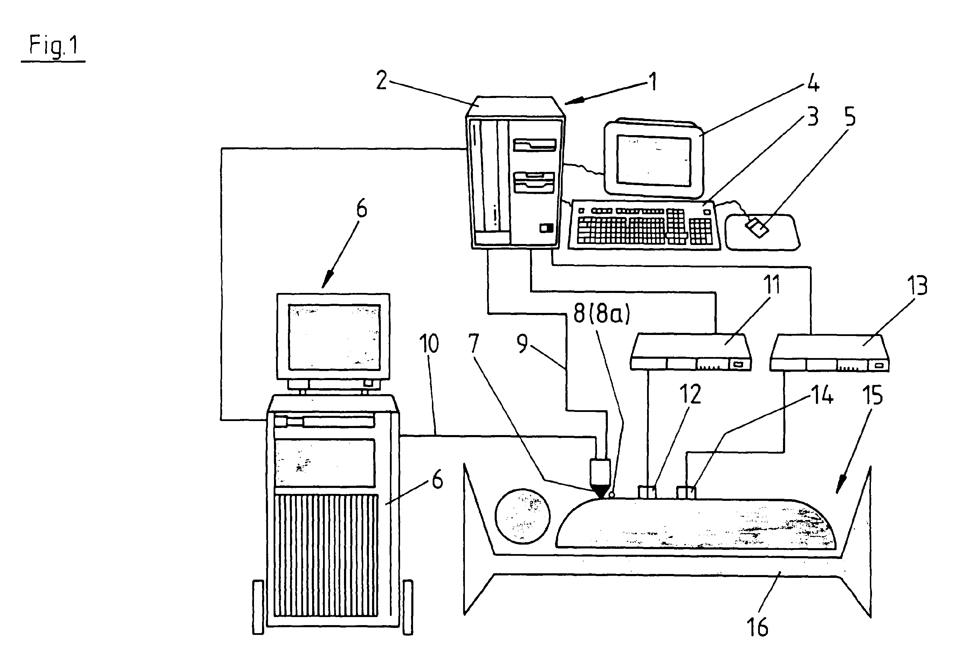

- Fig. 1: in schematischer Darstellung eine Ausführung des erfindungsgemäßen Systems zur Generierung von diagnostisch verwertbaren dreidimensionalen Datensätzen für eine tomographische Bilderfassung eines zu untersuchenden Volumens;
- Fig. 2 bis 4: jeweils in schematischer Darstellung verschiedene mögliche Ausführungen eines Wegaufnehmers zur Verwendung bei dem System der Figur 1;
- Fig. 5: in schematischer Darstellung die jeweils im Bildverarbeitungssystem abgelegten Einzelbilder, zusammen mit graphischen Darstellungen des EKG-Zyklus und des Respirations-Zyklus eines Patienten.

In den Figuren ist 1 ein Bildverarbeitungssystem, welches im wesentlichen aus dem Rechner 2 und aus den an diesem angeschlossenen peripheren Geräten, wie Tastatur 3, Bildschirm 4, Maussteuerung 5 usw. besteht. Dem Bildverarbeitungssystem sind zugeordnet:
Ein Ultraschallgerät 6 mit Ultraschallkopf 7, der von der untersuchenden Person frei bewegt bzw. geführt werden kann, und
ein elektrischer oder elektronischer Wegaufnehmer 8, 8a bzw. 8b an dem Ultraschallkopf.

Bei dem Ultraschallgerät 6 mit dem Ultraschallkopf 7 handelt es sich z.B. um ein dem Fachmann bekanntes und in der ärztlichen Diagnostik verwendetes Gerät zur Erfassung von Ultraschallbildern, die jeweils einen Bereich oder Schnitt des zu untersuchenden Volumens wiedergeben, der sich in der Bildebene des freigeführten Ultraschallkopfes 7 befindet.

Der Weggeber 8, 8a bzw. 8b ist bei der dargestellten Ausführungsform über eine Leitung 9 mit dem Rechner 2 verbunden, wobei diese Signalleitung 9 vorzugsweise in einem Kabel mit der Signalleitung 10 vorgesehen ist, die den Ultraschallkopf 7 mit dem Ultraschallgerät 6 verbindet. Grundsätzlich besteht auch die Möglichkeit, insbesondere anstelle der Leitung 9 eine drahtlose Übertragung der Signale des Weggebers 8, 8a bzw. 8b an den Rechner 2 beispielsweise über eine Funk- oder Infrarotstrecke vorzusehen.

Bei der dargestellten Ausführungsform sind dem Bildverarbeitungssystem weiterhin noch ein EKG-Gerät 11 mit zugehörigen Elektroden 12 und ein Gerät 13 zur Registrierung der Atemlage (Respirationszyklus) mit zugehörigen Sonden 14 zugeordnet. Mit 15 ist ein Patient bezeichnet, dessen Körper oder Körperbereich ein zu untersuchendes Volumen bildet. Der Patient ist auf einer Liege 16 angeordnet.

Der am Ultraschallkopf 7 vorgegebene Weggeber 8/8a/8b ist so ausgeführt, daß er bei Berührungskontakt an der Außenfläche des zu untersuchenden Volumens, d.h. an der Körperoberfläche des Patienten 15 ein Signal liefert, welches der Bewegung bzw. dem Weg des freigeführten Ultraschallkopfes 7 in wenigstens einer Bewegungsachse entspricht, die in einer Bewegungsebene liegt, welche mit der Ebene jedes von dem Ultraschallkopf gelieferten Einzelbildes einen vorgegebenen Winkel, beispielsweise einen Winkel von 90° einschließt.

Bei einer vereinfachten Ausführung besteht die Möglichkeit, daß der Wegaufnehmer 8 die Bewegung nur in einer Bewegungsachse der vorgenannten Bewegungsebene erfaßt. In diesem Fall ist die Ausbildung so getroffen, daß die Bewegungsachse senkrecht oder quer zur Bildebene der Einzelbilder liegt.

Grundsätzlich besteht aber auch die Möglichkeit, den Weggeber so auszuführen, daß er eine vollständigere Positions- und Orientierungsinformation liefert, d.h. nicht nur die Translation in einer Achse der Bewegungsebene, sondern in jeder Achsrichtung dieser Ebene.

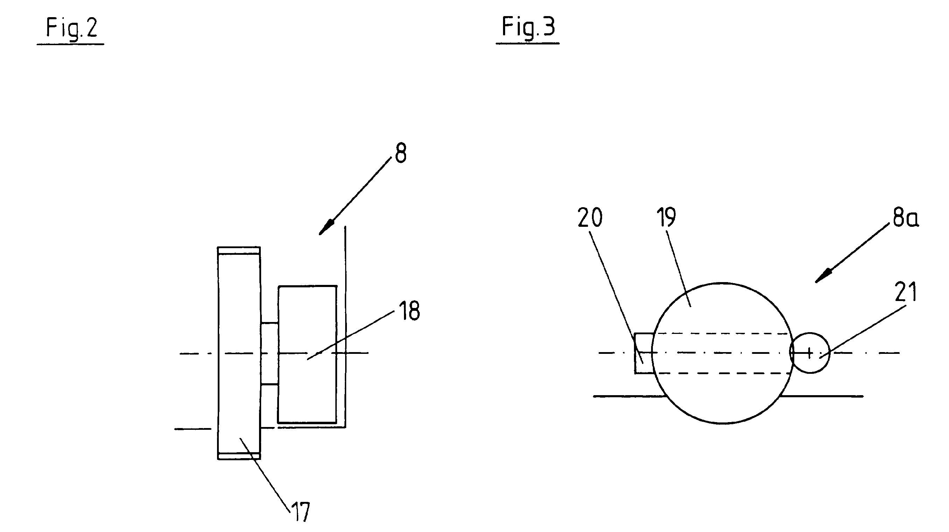

Wie in der Figur 2 dargestellt, besteht der Wegaufnehmer 8 beispielsweise aus einem kleinen Rad 17, welches an einem Wandler 18 vorgesehen ist, der die Drehung des Rades 17 in ein dieser Drehung proportionales Signal umwandelt. Der Wandler 18 ist beispielsweise ein analoger Winkelsensor, z.B. Potentiometer, dessen Ausgangsspannung über einen Analog-Digital-Wandler digitalisiert und über eine serielle Schnittstelle dem Rechner 2 zugeführt wird. Bei bekannter Eingangsspannung des Winkelsensors und bei bekanntem Umfang des Rades 17 kann die Translation der Verschiebung des Schallkopfes 7 in der entsprechenden Bewegungsachse ermittelt werden. Die Drehachse des Rades 17 liegt hierbei parallel zu einer Achse der Bildebene der jeweiligen, vom Ultraschallkopf erzeugten Einzelbilder.

Um mittels des Rechners 2 aus den Einzelbildern des Ultraschallkopfes 7 und dem Signal des Weggebers 8 einen Volumendatensatz zu generieren wird dann so verfahren, daß der Ultraschallkopf 7 am Beginn der Untersuchung, zumindest aber am Beginn der Aufnahme der Einzelbilder, aus denen der Volumendatensatz generiert werden soll, so orientiert ist, daß er in einem vorgegebenen Winkel, beispielsweise senkrecht auf der Oberfläche des zu untersuchenden Volumens aufsteht, diese Orientierung auch beim Weiterbewegung des Ultraschallkopfes 7 entlang der Oberfläche des zu untersuchenden Volumens aufrechterhalten wird und die Bewegungsrichtung hierbei möglichst senkrecht zur Achse des Rades 17 beibehalten wird. Während der Bilderfassung erfolgt nur eine Translation und keine Verkippung des Ultraschallkopfes 7.

Anstelle des analogen Winkelsensors kann auch ein anderer Sensor 18 vorgesehen sein, der in Abhängigkeit vom Drehen des Rades 17 ein Signal liefert, beispielsweise einen Sensor zur Erzeugung eines digitalen Signales.

Die Figur 3 zeigt in vereinfachter Darstellung den Wegaufnehmer 8a, der anstelle des Wegaufnehmers 8 verwendet werden kann und in der Lage ist, die beiden senkrecht zueinander orientierten Komponenten der jeweiligen Bewegung in der Bewegungsebene und damit die Richtung und den Weg der Bewegung in dieser Bewegungsebene zu erfassen. Der Weggeber 8a ist ähnlich einer Computer-Maus mit einer Rollkugel 19 ausgebildet, die zwei kleine Zylinder 20 und 21 antreibt, deren Drehachsen senkrecht zueinander stehen und in der Bewegungsebene liegen. Die Drehung der Zylinder wird in entsprechende digitale Signale umgewandelt und als Weggebersignal dem Rechner 2 zugeführt. Der Weggeber 8a ist dann beispielsweise über eine Halterung derart am Ultraschallkopf 7 befestigt, daß sich die Rollkugel 19 beim Verschieben des Ultraschallkopfes 7 auf der Oberfläche des zu untersuchenden Volumens auf dieser Oberfläche abrollt und die beiden Achsen der Zylinder 20 und 21 in der Bewegungsebene liegen, und zwar beispielsweise derart, daß eine der beiden Achsen parallel zu einer Achsrichtung, nämlich zur horizontalen Achsrichtung der Bildebene des jeweiligen Einzelbildes liegt, während die Achse des anderen Zylinders quer oder senkrecht zu dieser Bildebene orientiert ist.

Um aus den Einzelbildern und den Daten des Weggebers 8a einen Volumendatensatz zu erstellen, ist es auch bei Verwendung des Weggebers 8a notwendig, daß zumindest am Beginn der Aufnahme der für den Volumendatensatz verwendeten Einzelbilder und während dieser Aufnahme eine vorgegebene Orientierung des Ultraschallkopfes 7 in Bezug auf die Oberfläche des zu untersuchenden Volumens eingehalten und aufrecherhalten wird, beispielsweise eine senkrechte Orientierung. Bei der Bilderfassung ist somit wiederum nur eine Translation des Ultraschallkopfes 7 möglich, aber kein Verkippen des Ultraschallkopfes 7.

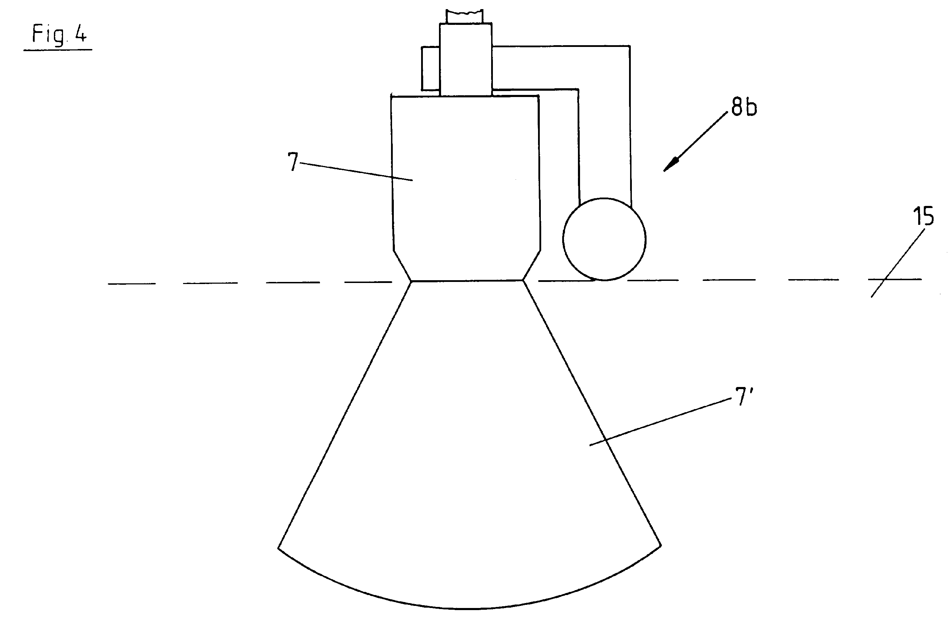

Die Figur 4 zeigt in vereinfachter Darstellung und in Seitenansicht nochmals den Ultraschallkopf 7, wobei mit 7' auch die Bildebene des Ultraschallkopfes angedeutet ist, die parallel zur Zeichenebene der Figur 4 liegt. An dem Ultraschallkopf 7 ist ein Wegaufnehmer 8b vorgesehen, der ähnlich dem Wegaufnehmer 8 ausgebildet ist und wiederum ein Rad 17 aufweist, welches mit einem in der Figur 4 nicht dargestellten Wandler 18 zusammenwirken. Abweichend von dem Wegaufnehmer 8 ist der Wegaufnehmer 8b so orientiert, daß die Achse des Rades 17 senkrecht zur Bildebene der vom Ultraschallkopf 7 erzeugten Einzelbilder liegt.

Mit dem Weggeber 8b werden also Weg-Daten erzeugt, die der Bewegung des Ultraschallkopfes 7 parallel zu seiner Bildebene 7' entsprechen der Weggeber 8b eignet sich insbesondere auch zur Verwendung in Kombination mit dem Weggeber 8, um so Weg-Daten zu erhalten, die einer Bewegung in einer Ebene senkrecht zur Bildebene 7' entsprechen.

Das EKG-Gerät 11 ist ein solches, welches dem Fachmann aus der ärztlichen Praxis und Diagnostik bekannt ist und welches in Abhängigkeit von dem EKG-Zyklus an einem Ausgang eine sich ändernde Spannung liefert. Bei dem Gerät 13 handelt es sich ebenfalls um ein dem Fachmann bekanntes Gerät, welches in der ärztlichen Praxis bzw. Diagnostik zur Erfassung des Respirationszyklus verwendet wird und in Abhängigkeit von diesem Respirationszyklus am Ausgang eine sich ändernde Spannung liefert.

Bei der Aufnahme des dreidimensionalen Datensatzes wird folgendermaßen verfahren:
Die Ultraschall-Einzelbilder werden vom Rechner 2 des Bildverarbeitungssystems 1 erfaßt. Dann, wenn das Ultraschallgerät 6 analoge Bilder liefert, werden diese digitalisiert. Liefert das Ultraschallgerät 6 bereits Einzelbilder in digitaler Form, so können diese ohne Umwandlung im Rechner 2 erfaßt und verarbeitet werden.

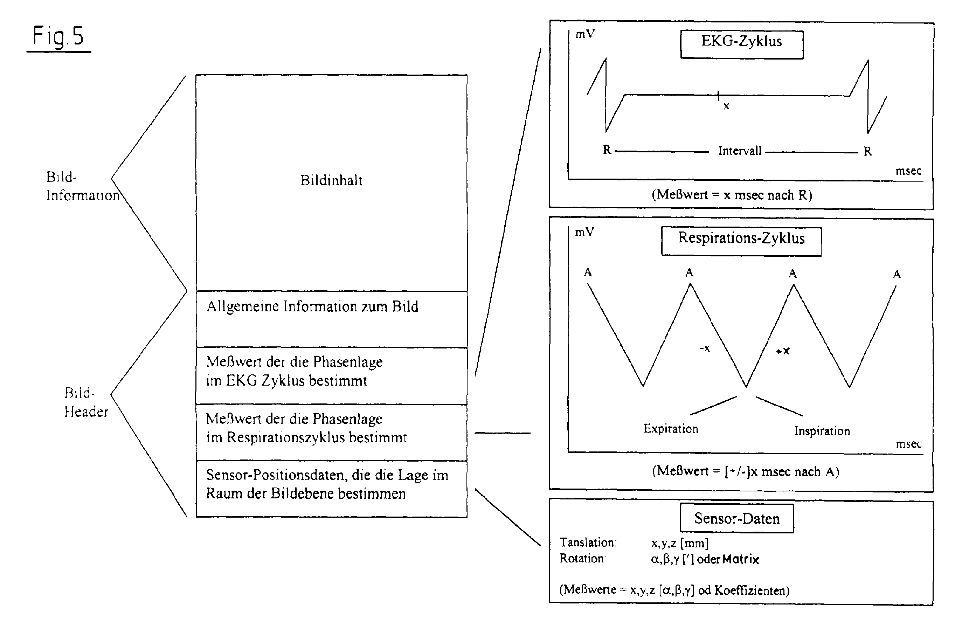

Im Rechner 2 wird weiterhin zu jedem Einzelbild auch das Signal des Weggebers 8 bzw. 8a erfaßt und in einem dem jeweiligen Einzelbild zugeordneten Datensatz, beispielsweise in einem Bildheader des Datensatzes abgespeichert, der diesen Bildheader und den Bildinhalt enthält. Dies erfolgt z.B. "online", d.h. sofort bei der Bild- oder Datenaquisition oder aber nach der Bild- oder Datenaquisition in einem nachfolgenden ersten Schritt der Nachbearbeitung. Der Bildheader enthält beispielsweise auch Werte des EKG-Gerätes 11, z.B. einen Meßwert, der die Phasenlage des jeweiligen Einzelbildes in Bezug auf den EKG-Zyklus bestimmt, und/oder z.B. einen Meßwert des Gerätes 13, der beispielsweise die Phasenlage des Einzelbildes in Bezug auf den Respirationszyklus bestimmt, wie dies in der Figur 5 dargestellt ist.

Mit Hilfe der Signale des Weggebers 8, 8a bzw. 8b wird die Erfassung der Einzelbilder (Bild- oder Datenaquisition) durch das System 1 bzw. den Rechner 2 so gesteuert, daß von dem Ultraschallgerät 6 gelieferte Einzelbilder nur dann akzeptiert bzw. dann abgespeichert werden, wenn der Ultraschallkopf 7 über eine, der jeweils gewünschten und vorzugsweise im System einstellbaren oder vorwählbaren Auflösung entsprechenden Wegstrecke bewegt wurde.

Die von dem Weggeber 8, 8a bzw. 8b gelieferten Signale werden vom Rechner 2 ständig erfaßt und verarbeitet, um insbesondere aus diesen Signalen zu jedem Zeitpunkt die Lage des Ultraschall-Kopfes 7 bezogen auf eine Ausgangsposition zu bestimmen.

Bei Verwendung des Weggebers 8, der die Translation nur in einer Achsrichtung liefert, wird bei der Ermittlung der Lage und Position des Ultraschallkopfes im Rechner 2 davon ausgegangen, daß der Ultraschall-Kopf 7 bei der Aufnahme der Einzelbilder nur in dieser Bewegungsachse senkrecht oder quer zu der Bildebene geradlinig bewegt wurde.

Bei Verwendung des Weggebers 8a oder der Kombination der Weggeber 8 und 8b werden aus den von diesem Weggeber gelieferten Signalen zu jedem Zeitpunkt die Lage und auch tatsächliche Orientierung des Ultraschallkopfes 7 unter Berücksichtigung der Vorschriften zum Führen dieses Ultraschallkopfes (nur Translationsbewegungen - keine Kipp- oder Drehbewegungen) ermittelt.

Die Zuordnung der Ultraschall-Einzelbilder und der Lage- und Positionsdaten erfolgt z.B. über einen Arbeits- oder Zeittakt des Rechners 2.

Die im Rechner 2 abgespeicherten Bilddaten mit zugehörigem Bildheader bilden einen Rohdatensatz, in dem inbesondere bei Verwendung des Weggebers 8a die Bildebenen der Einzelbilder unterschiedlich orientiert sein können. Aus diesem Rohdatensatz wird dann durch ein Transformations- und/oder Interpolationsverfahren der diagnostisch verwertbare dreidimensionale Datensatz in einem einheitlichen Bezugs- oder Koordinatensystem erstellt. Hierfür kann in einer in der EP 0 865 765 detailliert beschriebenen Weise vorgegangen werden.

Durch das EKG-Gerät 11 und das Gerät 13 besteht die Möglichkeit dynamische, aber auch statische dreidimensionale Datensätze zu generieren, und zwar in Bezug auf den Herzzyklus bzw. die Atmung des Patienten 15. Insbesondere besteht die Möglichkeit einer EKG-Triggerung bei der Bild- oder Datenaquisition durch den Rechner 2.

## Patentansprüche

1. Verfahren zur Generierung eines diagnostisch verwertbaren dreidimensionalen Bilddatensatzes unter Verwendung eines Ultraschallgerätes (6) mit frei geführtem Ultraschallkopf (7) zur Erzeugung einer Sequenz von Ultraschalleinzelbildern in unterschiedlichen Ebenen des zu untersuchenden Volumen, unter Verwendung einer Bildverarbeitungseinrichtung (1), der die Ultraschalleinzelbilder zugeführt werden, unter Verwendung wenigstens eines Positions-Sensors (8, 8a, 8b), welcher zumindest die Position des Ultraschall-Kopfes und damit die Lage der Bildebene des jeweils generierten Ultraschallbildes bestimmt und dessen Daten ebenfalls an die Bildverarbeitungseinrichtung (1) übertragen werden, die aus den Bilddaten der Ultraschall-Einzelbilder und den Positions-Daten den dreidimensionalen, das untersuchte Volumen tomographisch erfassenden Datensatz generiert, **dadurch gekennzeichnet**,
daß das Positions-Sensorelement ein am Ultraschallkopf (7) angebrachter Wegaufnehmer (8, 8a, 8b) ist, der mit dem Ultraschallkopf (7) entlang der Oberfläche des zu untersuchenden Volumens und an dieser Oberfläche anliegend bewegt wird.

2. Verfahren nach Anspruch 1, gekennzeichnet durch die Verwendung wenigstens eines nur die Translations-Bewegung in einer Achsrichtung erfassenden Wegaufnehmers (8, 8b).

3. Verfahren nach Anspruch 2, gekennzeichnet durch die Verwendung eines die Translations-Bewegung in einer Achsrichtung quer oder senkrecht zur Bildebene des Ultraschallkopfes (7) erfassenden Wegaufnehmers (8).

4. Verfahren nach Anspruch 2 oder 3, gekennzeichnet durch die Verwendung eines die Translations-Bewegung in einer Achsrichtung parallel oder etwa parallel zur Bildebene des Ultraschallkopfes (7) erfassenden Wegaufnehmers (8b).

5. Verfahren nach einem der Ansprüche 1 bis 4, gekennzeichnet durch die Verwendung eines die Translationsbewegung in zwei senkrecht zueinander verlaufenden Bewegungsrichtungen erfassenden Wegaufnehmers (8a).

6. System zur Generierung eines diagnostisch verwertbaren dreidimensionalen Bilddatensatzes mit einem Ultraschallgerät (6) mit frei geführtem Ultraschallkopf (7) zur Erzeugung einer Sequenz von Ultraschalleinzelbildern in unterschiedlichen Ebenen des zu untersuchenden Volumen, mit einer Bildverarbeitungseinrichtung (1), der die Ultraschalleinzelbilder zugeführt werden, mit wenigstens einem Positions-Sensor (8, 8a, 8b), welcher zumindest die Position des Ultraschall-Kopfes und damit die Lage der Bildebene des jeweils generierten Ultraschallbildes bestimmt und dessen Daten ebenfalls an die Bildverarbeitungseinrichtung (1) übertragen werden, die aus den Bilddaten der Ultraschall-Einzelbilder und den Positions-Daten den dreidimensionalen, das untersuchte Volumen tomographisch erfassenden Datensatz generiert, **dadurch gekennzeichnet**, daß das Positions-Sensor ein am Ultraschallkopf (7) angebrachter Wegaufnehmer (8, 8a, 8b) ist, der mit dem Ultraschallkopf (7) entlang der Oberfläche des zu untersuchenden Volumens und an dieser Oberfläche anliegend bewegt wird.

7. System nach Anspruch 6, gekennzeichnet durch wenigstens einen nur die Translations-Bewegung in einer Achsrichtung erfassenden Wegaufnehmer (8, 8b).

8. Verfahren nach Anspruch 7, gekennzeichnet durch einen die Translations-Bewegung in einer Achsrichtung quer oder senkrecht zur Bildebene des Ultraschallkopfes (7) erfassenden Wegaufnehmer (8).

9. Verfahren nach Anspruch 7 oder 8, gekennzeichnet durch einen die Translations-Bewegung in einer Achsrichtung parallel oder etwa parallel zur Bildebene des Ultraschallkopfes (7) erfassenden Wegaufnehmer (8b).

10. System nach einem der Ansprüche 6 bis 9, gekennzeichnet durch einen die Translationsbewegung in zwei senkrecht zueinander verlaufenden Bewegungsrichtungen erfassenden Wegaufnehmer (8a).
